# EUROPEAN PATENT APPLICATION

(11) **EP 3 708 006 A1**
(43) Date of publication of application: **16.09.2020**
(21) Application number: 19425017.1
(22) Date of filing: 14.03.2019
(51) Int. Cl.: A23B 7/148

(54) **METHOD OF CONTROLLING CONTROLLED ATMOSPHERE CELLS FOR STORING PERISHABLE ITEMS**

(71) Applicant: Isolcell S.p.A., 39055 Laives (Bolzano) (IT)
(72) Inventor: Pruneri, Giorgio, 39100 Bolzano (IT)
(74) Representative: Forattini, Amelia

(57) **Abstract**

A control method for controlled atmosphere cells for storing perishable items, for use in a cell containing a product to be stored by means of control means for controlling the atmosphere inside the cell, said control means comprising at least a CO₂ extraction system and an O₂ variation system; said control means being controlled by a management system; said management system providing a level of storage oxygen to said control means; the method comprises: a first measurement of the value of O₂ and CO₂ in a storage cell containing items to be stored; a second measurement of the value of CO₂ performed after a certain time with respect to said first measurement; a calculation of the new level of the storage oxygen to be set in the management system on the basis of the increase of the value of CO₂ detected in said second measurement; a repetition, at preset time intervals, of said first and said second measurement and of said calculation in order to determine each time a new level of storage oxygen to be set in the management system. The method according to the invention is based on determining the Lowest Oxygen Limit only by the determination of the increase of CO₂ produced by fruits or vegetables in a certain period of time, thus completely eliminating the problem concerning the difficulty of correctly measuring the value of the oxygen used by the stored product.

## Description

The present invention relates to a control method for controlled atmosphere cells for storing perishable items.

As is known, perishable items such as fruit and vegetables are often stored and transported in controlled atmosphere cells, known as "CA cells", in which the temperature and atmospheric composition can be controlled in order to prolong the life of the products.

CA cells are normally gas-tight and are provided with systems for monitoring and controlling the temperature and the levels of oxygen, carbon dioxide and nitrogen.

Atmosphere control systems generally operate by repeatedly sampling the gas levels within the cell and by adding or removing the gases in order to keep the atmosphere at the desired values.

The reference values are generally predetermined on the basis of the knowledge acquired in previous experiences with the stored products.

For example, a set of oxygen reference values can be developed for a specific fruit in order to allow the system to adjust the oxygen levels in the atmosphere and follow a preset profile over time. However, those reference values can vary significantly due to various factors.

For the above reason, controlled atmosphere cells are used which are provided with control systems capable of performing a dynamic control of the atmosphere, so-called DCA (Dynamic Controlled Atmosphere) cells.

DCA technology allows to dynamically control the atmosphere in a CA cell not on the basis of predetermined reference values but rather on the basis of information that originates from controlling the response of the products to environmental changes.

The response of the stored goods to changes can be determined by characteristics affected by metabolic processes, such as the production of ethanol, chlorophyll fluorescence, and respiration.

One of the most important factors that relate to the atmosphere is respiration.

The respiration of fruit and vegetables can have a significant effect on the levels of oxygen and carbon dioxide in a cell.

Respiration of fruit and vegetables is the natural ripening process that occurs after picking.

Respiration is the absorption of oxygen and the emission of carbon dioxide, just like the human body does during breathing.

It is known that the storage of fruits and vegetables in controlled atmosphere is more advantageous when the oxygen level inside the storage chamber is low; however, it is also known that there is a limit to said lowering, because too low oxygen levels can lead to the fermentation of the product.

In order to set said limit, known as L.O.L. (Lowest Oxygen Limit), a conventional method determines the respiratory quotient (RQ), that is the ratio between the quantity of carbon dioxide (CO₂) produced by fruits (or by vegetables) and the quantity of oxygen (O₂) used by the same in a predetermined period. To this end, the values of CO₂ and O₂ inside the storage cell are measured a first time and then a second time, after a few hours. In the period between the two measurements, the apparatuses that can cause interferences in the determination of the values of CO₂ and O₂, must be stopped, such as for example CO₂ absorbers, the refrigeration unit, the oxygen input apparatus.

Various systems for controlling the respiration quotient (RQ) within a dynamically controlled atmosphere cell are known for knowing the actual variations of oxygen and carbon dioxide inside said cell.

RQ measurement can be difficult because of the uncertainty of the gas tightness of the cell and due to the addition of N₂ gas, in order to reduce the oxygen, or the removal of CO₂ gas from external scrubbing systems, which are normal processes associated with the management of many CA cells.

US 2012/0097050 discloses a system, for dynamic control of the atmosphere in a cell for the storage of perishable items such as fruit and vegetables, that uses a small chamber that is arranged inside the storage cell and in which there is a sample of the items. The chamber remains temporarily closed, without any gas flow, during RQ measurement and for this reason the consumption of oxygen is rapid and total, i.e., it is consumed in 12 hours or less, while part of the CO₂ that has formed by anaerobic respiration does not have enough time to exit from the pulp, and this causes an error in the calculation of RQ. CO₂ does not have enough time to escape because of the high solubility of CO₂ in cellular sap.

Another drawback of the system described in US2012/0097050 is constituted by the fact that it uses a flow of nitrogen, in order to eliminate CO₂ when it reaches high values. Due to the high generation cost of nitrogen, the system proposed by US 2012/0097050 is economically disadvantageous.

A further drawback of the system described in US2012/0097050 is constituted by the fact that an imperfect seal of the gas analyzer or of the suction and return pipes can cause the introduction of O₂ in the chamber during RQ determination, inducing errors in its calculation.

Another problem of the system described in US2012/0097050 is that the measurement chamber is sealed by means of water, which can alter the internal humidity of the chamber, or can create an environmental condition that is excessively humid with respect to the condition of the CA cell. Also, in case of evaporation of the water, in order to ensure tightness, it would be necessary to top up the water periodically in the sealing channel; however, this addition of water is complicated, because access to the storage cell by personnel is restricted because of the lethal concentration of gas in the CA cell.

Another problem of US2012/0097050 is constituted by the fact that any inclination of the cell might cause the escape of water from the sealing channel and therefore the possible entry of gas of the CA cell, thus altering RQ determination. Also, the CO₂ inside the chamber can be solubilized in the water of the sealing channel, altering the content of CO₂ in the atmosphere and generating errors in RQ calculation.

WO2013125944 discloses a method and an apparatus for controlling the atmosphere in a cell that contains horticultural products, by means of a determination of RQ in the atmosphere of the entire CA cell. The system described in WO2013125944 requires an almost complete seal of the CA cell; however, the seal of commercial CA cells decreases as the operating time increases and also there are great differences in tightness among the different CA cells. Also, the system described in WO2013125944 requires disconnection from the refrigeration system and from the gas control system of the CA cell for 4 hours, with the consequent drawback of the temporary rise of the temperature of the fruits stored in the CA cell. A further drawback of the system described in WO2013125944 is that it requires measurement of the internal pressure of the CA cell and pressurization of the latter with nitrogen during QR determination, with consequent additional costs for the production of nitrogen and for the system for measuring and adjusting the pressure of the CA cell. Another drawback of the system described in WO2013125944 resides in that an error of the control system might cause an excessive stress to all the fruit stored in the CA cell if RQ measurement is not interrupted after the 4 hours planned for this operation.

WO2011113915 discloses a storage system in which RQ determination occurs in the atmosphere of the entire CA cell. In the system described in WO2011113915, the O₂ level is established as a function of the GERQ (Gas Exchange Rate Quotient) and of the inflow of air in the CA cell. The GERQ and the inflow of air in the CA cell are calculated by means of mathematical models that simulate these phenomena. The GERQ is calculated by using the respiratory quotient and the diffusion of the gases through the pulp of the fruit. The purpose of WO2011113915 is to determine the beginning of anaerobic respiration, in order to avoid it with an increase in the level of oxygen inside the storage cells, so as to maintain only normal (aerobic) respiration with an RQ around 1.0. The system disclosed in WO2011113915 requires the complete tightness of the CA cell. This tightness is calculated on the basis of a mathematical model that establishes the O₂ setpoint; however, since the variability of the gas tightness of a commercial CA cell is not predictable, the mathematical model can be subject to errors. The tightness of the cells varies as a function of damage to panels and doors, aging of the waterproofing materials, age of the cells, imperfect door closure, movement and cracking of the floor are some among the causes that are difficult to predict. Also, the system described in WO2011113915 requires the injection of nitrogen (scrubbing) for the CO₂ absorber.

As described above, it is difficult to measure the quantity of oxygen consumed by the item within a storage cell because, despite the tightness of the cell, there is always a certain residual permeability to the oxygen present outside the cell.

EP2918179 solves this problem by means of tight containers that are located inside the cell and in each of them a representative sample of the good that is stored in the cell, is inserted.

The aim of the present invention is to provide a new control method for controlled atmosphere cells, for storing perishable items, that overcomes the drawbacks of the cited prior art.

An important object of the invention is to provide a method that allows to overcome the problem concerning the difficulties of correctly measuring the value of the oxygen that is consumed by the product being stored.

Another object of the present invention is to provide a method which, thanks to its peculiar characteristics of application, is able to offer the best guarantee of reliability and safety in use.

This aim, these objects and others that will become better apparent hereinafter are achieved by a control method for controlled atmosphere cells for storing perishable items, for use in a cell containing a product to be stored by means of control means for controlling the atmosphere inside the cell, said control means comprising at least a CO₂ extraction system and an O₂ variation system; said control means being controlled by a management system; said management system providing a level of storage oxygen to said control means; said method being characterized in that it comprises:
- a first measurement of the value of O₂ and CO₂ in a storage cell containing items to be stored;
- a second measurement of the value of CO₂ performed after a certain time with respect to said first measurement;
- a calculation of a new level of the storage oxygen to be set in the management system on the basis of the increase of the value of CO₂ detected in said second measurement;
- a repetition, at preset time intervals, of said first and said second measurement and of said calculation in order to determine each time a new level of storage oxygen to be set in the management system.

The method according to the present invention is advantageously used in storage cells for foodstuff, such as fruits and vegetables.

The storage cell may have any volume and does not need a total tightness, as long as it allows the maintenance of low concentrations of O₂ , such as for example at 0,2%.

The cell preferably comprises an absorber of CO₂ with an automatic system for the injection of O₂, a refrigeration plant and a plenum chamber to balance the pressure variations in the cell.

According to the present invention, the Lowest Oxygen Limit (LOL) is defined only by the determination of the increase of CO₂ produced by fruits or vegetables contained in the cell in a certain period of time.

The control method comprises the following steps.

A first step comprises the measurement of the value of O₂ and CO₂ in the storage cell; said measurement can be performed by apparatuses of a known type, for example by a Multiplex analyzer, manufactured by firm Isolcell.

A second step comprises stopping of the functioning of the apparatuses of the cell for a few hours, particularly of the apparatuses which may influence the measurement of the values of CO₂, such as for example the CO₂ extraction system.

Said second step is managed by the plant management system, for example by the software ISOSOFT, by firm Isolcell.

A third step comprises a new measurement of CO₂.

A fourth step comprises the calculation, by means of an adapted algorithm, of the new storage oxygen level to be set in the plant management system on the basis of the increase of the value of CO₂ detected in the third step.

The method includes the repetition of the first, second, third and fourth step, that is of the calculation of the increase of the level of CO₂ in a certain period of time and of the calculation of the fresh level of storage oxygen to be set at time intervals which may vary according to the control frequency one wishes to follow.

In practice it has been found that the invention achieves the intended aim and objects, providing a new control method that allows to determine the lowest level of oxygen, within a controlled atmosphere cell, by means of structures and apparatuses that are substantially simpler and cheaper with respect to the systems of the prior art.

The method according to the invention is based on determining the Lowest Oxygen Limit only by virtue of the determination of the increase of CO₂ produced by fruits or vegetables in a certain period of time, thus completely eliminating the problem concerning the difficulty of correctly measuring the value of the oxygen used by the stored product.

## Claims

1. A control method for controlled atmosphere cells for storing perishable items, for use in a cell containing a product to be stored by means of control means for controlling the atmosphere inside the cell, said control means comprising at least a CO₂ extraction system and an O₂ variation system; said control means being controlled by a management system; said management system providing a level of storage oxygen to said control means; said method being **characterized in that** it comprises:
- a first measurement of the value of O₂ and CO₂ in a storage cell containing items to be stored;
- a second measurement of the value of CO₂ performed after a certain time with respect to said first measurement;
- a calculation of a new level of the storage oxygen to be set in the management system on the basis of the increase of the value of CO₂ detected in said second measurement;
- a repetition, at preset time intervals, of said first and said second measurement and of said calculation in order to determine each time a new level of storage oxygen to be set in the management system.

2. The method, according to claim 1, **characterized in that** between said first and said second measurement, said management system orders the temporary stop of the functioning of said CO₂ extraction system.

3. The method, according to claim 1, **characterized in that** said temporary stop is in the order of a few hours.
